# EUROPEAN PATENT APPLICATION

(11) **EP 4 019 050 A1**
(43) Date of publication of application: **29.06.2022**
(21) Application number: 20853969.2
(22) Date of filing: 19.08.2020
(51) Int. Cl.: A61K 47/68, A61K 38/21, A61P 35/00, C07K 14/565, C07K 16/32, A61K 39/00

(54) **USE OF IMMUNOCYTOKINE COMPRISING INTERFERON-BETA OR VARIANT THEREOF FOR TREATING HUMAN EPIDERMAL GROWTH FACTOR RECEPTOR 2 POSITIVE CANCER**

(30) Priority: 19.08.2019 KR 20190101351
(71) Applicant: GenoPharm Inc., Seoul 08394 (KR)
(72) Inventor: JEONG, Hae Min, Seoul 08394 (KR); LEE, Ji Sun, Seoul 08394 (KR)
(74) Representative: Richardt Patentanwälte PartG mbB
(86) International application number: PCT/KR2020/011030
(87) International publication number: WO 2021/034098

(57) **Abstract**

The present invention relates to a use of a recombinant protein in which an interferon-beta protein and an antibody binding to a HER2 antigen are fused for the purpose of treating cancer patients of which the HER2 expression level is IHC 1+ or higher. The recombinant protein can exhibit efficacy better than conventional antibody therapeutic agents through cancerspecific anti-cancer immune responses in patients, thereby being used for more patients, and thus is effectively usable as a novel agent for treating cancer.

<110>    GenoPharm Inc.

<120>    Use of Interferon-beta mutein Immunocytokines for Treatment of
         Human Epidermal Growth Factor Receptor 2 Positive Cancer

<130>    OP20-0045/PCT

<150>   KR 10-2019-0101351
<151>   2019-08-19

<160>    5

<170>    KoPatentIn 3.0

<210>    1
<211>    166
<212>    PRT
<213>    Artificial Sequence

<220>
<223>    Amino acid sequence of interferon beta mutein


<400>    1
Met Ser Tyr Asn Leu Leu Gly Phe Leu Gln Arg Ser Ser Asn Phe Gln
  1               5                  10                  15

Cys Gln Lys Leu Leu Trp Gln Leu Asn Gly Thr Leu Glu Tyr Cys Leu
             20                  25                  30

Lys Asp Arg Met Asn Phe Asp Ile Pro Glu Glu Ile Lys Gln Leu Gln
         35                  40                  45

Gln Phe Gln Lys Glu Asp Ala Ala Leu Thr Ile Tyr Glu Met Leu Gln
     50                  55                  60

Asn Ile Phe Ala Ile Phe Arg Gln Asp Ser Ser Ser Thr Gly Trp Asn
 65                  70                  75                  80

Glu Thr Ile Val Glu Asn Leu Leu Ala Asn Val Tyr His Gln Ile Asn
                 85                  90                  95

His Leu Lys Thr Val Leu Glu Glu Lys Leu Glu Lys Glu Asp Phe Thr
            100                 105                 110

Arg Gly Lys Leu Met Ser Ser Leu His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165


<210>    2
<211>    501
<212>    DNA
<213>    Artificial Sequence

<220>
<223>    Polynucleotide sequence of interferon beta mutein


<400>    2
atgtcctaca acctgctggg cttcctgcag cggtcctcca acttccagtg ccagaaactg          60

ctgtggcagc tgaatggcac cctggaatac tgcctgaagg accggatgaa cttcgacatc         120

cccgaggaaa tcaagcagct gcagcagttc cagaaagagg acgccgctct gaccatctac         180

gagatgctgc agaacatctt cgccatcttc cggcaggact cctcctccac cggctggaac         240

gagacaatcg tggaaaatct gctggccaac gtgtaccacc agatcaacca cctgaaaacc         300

gtgctggaag agaagctgga aaaagaggac ttcacccggg gcaagctgat gtcctccctg         360

cacctgaagc ggtactacgg ccggatcctg cactacctga aggccaaaga atactcccac         420

tgcgcctgga ccatcgtgcg ggtggaaatc ctgcggaact tctactttat caaccggctg         480

accggctacc tgaggaactg a                                                   501


<210>    3
<211>    450
<212>    PRT
<213>    Artificial Sequence

<220>
<223>    Amino acid sequence of Trastuzumab heavy chain


<400>    3
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450


<210>    4
<211>    214
<212>    PRT
<213>    Artificial Sequence

<220>
<223>    Amino acid sequence of Trastuzumab light chain


<400>    4
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>    5
<211>    631
<212>    PRT
<213>    Artificial Sequence

<220>
<223>    Amino acid sequence of interferon beta mutein-Trastuzumab heavy
         chain


<400>    5
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys Gly Gly Gly Gly Ser Gly Gly Gly Gly Ser Gly Gly Gly Ser
    450                 455                 460

Gly Met Ser Tyr Asn Leu Leu Gly Phe Leu Gln Arg Ser Ser Asn Phe
465                 470                 475                 480

Gln Cys Gln Lys Leu Leu Trp Gln Leu Asn Gly Thr Leu Glu Tyr Cys
                485                 490                 495

Leu Lys Asp Arg Met Asn Phe Asp Ile Pro Glu Glu Ile Lys Gln Leu
            500                 505                 510

Gln Gln Phe Gln Lys Glu Asp Ala Ala Leu Thr Ile Tyr Glu Met Leu
        515                 520                 525

Gln Asn Ile Phe Ala Ile Phe Arg Gln Asp Ser Ser Ser Thr Gly Trp
    530                 535                 540

Asn Glu Thr Ile Val Glu Asn Leu Leu Ala Asn Val Tyr His Gln Ile
545                 550                 555                 560

Asn His Leu Lys Thr Val Leu Glu Glu Lys Leu Glu Lys Glu Asp Phe
                565                 570                 575

Thr Arg Gly Lys Leu Met Ser Ser Leu His Leu Lys Arg Tyr Tyr Gly
            580                 585                 590

Arg Ile Leu His Tyr Leu Lys Ala Lys Glu Tyr Ser His Cys Ala Trp
        595                 600                 605

Thr Ile Val Arg Val Glu Ile Leu Arg Asn Phe Tyr Phe Ile Asn Arg
    610                 615                 620

Leu Thr Gly Tyr Leu Arg Asn
625                 630

## Description

### FIELD OF THE INVENTION

The present application claims a priority of the Korean patent application No.10-2019-0101351 filed on August 19, 2019, and its entire specification is cited herein as a part of the specification of the present application.

The present invention relates to a use of a recombinant protein in which an interferon-beta and a human epidermal growth factor receptor 2 (HER2) are connected to each other for treating cancer, and more specifically to a use of a recombinant protein in which an interferon or a variant of the interferon-beta and an anti-HER2 antibody are connected to each other for treating cancer with the HER2 expression level of IHC 1+ or higher.

### BACKGROUND OF THE INVENTION

Interferon (IFN) is one of the major cytokines that play an important role in immunity, and is known to have a strong anti-cancer effect. These interferons are classified into type I (IFN-α and IFN-β), type II (IFN-γ), and type III (IFN-λ) .

Type I interferon exhibits anti-viral and anti-proliferative effects, and also plays an essential role in cancer immune-surveillance, the function of recognizing tumor-specific antigens and eliminating tumor cells. Among type I interferons, IFN-β has a stronger inhibitory effect on cell growth than IFN-α. In particular, when used together with an anti-cancer agent, the working range of anti-proliferative activity and synergistic effect of IFN-β are very good. However, it is known that the interferon treatment has high cytotoxicity which causes symptoms such as fever (80%), myalgia (73%), headache (50%), fatigue (50%), malaise (50%), etc. in the treatment recipients.

Trastuzumab is an antibody cancer treatment that targets human epidermal growth factor receptor 2 (HER2), and is being used to treat HER2-positive cancer patients. However, since trastuzumab alone is not sufficient to induce a therapeutic effect, pertuzumab, another antibody treatment targeting HER2, and chemotherapeutics are being administered in combination. In addition, among HER2-positive cancer patients, it is used only in a certain patient group with high levels of HER2 expression, and still there are limitations such as cancer recurrence or resistance to HER2 treatment after administration of the antibody treatment.

Specifically, therapeutic agents that have recently been used to treat HER2-positive cancer, such as trastuzumab and pertuzumab, are prescribed to obtain clinical responses only in patients who have malignant HER2-positive tumor cells with more than 1,000,000 HER2 receptors on the cell surface. Such patients having malignant HER2-positive tumor cells with more than 1,000,000 HER2 receptors on the cell surface are typically classified as HER2 IHC 3+. Alternatively, trastuzumab and pertuzumab can be prescribed to patients with the HER2 expression level of IHC 2+ together with the presence of HER2 gene amplification in a fluorescence *in situ* hybridization (FISH) assay, namely patients determined to be FISH positive by those skilled in the art.

Patients are classified based on their HER2 expression levels measured using, for example, HercepTest^{™} and/or HER2 FISH (pharm Dx^{™}) provided by Dako Denmark A/S, or HERmark^{®} assay provided by Monogram Biosciences. Patients with tumor cells with low HER2 expression levels, i.e., HER2 expression levels of IHC 1+, or IHC 2+ together with a FISH negative result, typically do not show sufficient clinical responses to trastuzumab and pertuzumab treatment. Therefore, trastuzumab and pertuzumab are limited in their use as cancer treatments since they are prescribed only to a specific range of patients.

On the other hand, signaling of type I interferon is a stepwise activation of a number of enzymes by the activity of the interferon receptor, in which case transcription factors such as STAT1 and STAT2 also participate. Such signaling activates the immune system, which is essential for numerous anti-cancer medicines to exert their anti-cancer effect, for example, chemotherapeutics such as anthracyclines, antibody treatments targeting growth factor receptors such as human epidermal growth factor receptor 2 (HER2), epidermal growth factor receptor (EGFR), etc. as well as adjuvants and oncolytic virotherapy treamtment. In particular, pSTAT3, one of the signaling molecules involved in the treatment resistance mechanism of trastuzumab, exists in a form in which two identical molecules are polymerized (pSTAT3 homodimer), and is known to be inhibited by pSTAT1. Therefore, enhanced anti-cancer efficacy can be expected when type I interferon activating pSTAT1 is combined with trastuzumab.

Accordingly, the present inventors have developed a new therapeutic agent with enhanced treatment efficacy compared to the existing HER2 antibody therapeutics by producing a recombinant protein in which an interferon-beta protein and a HER2-targeting antibody are fused together. Unlike the current HER2 antibody treatments, the fusion protein of the present invention shows treatment efficacy in patients with low HER2 expression levels, moreover, it can effectively lower the probability of cancer recurrence, suggesting a possibility of developing a novel cancer treatment.

### DESCRIPTION OF THE INVENTION

### PROBLEMS TO BE SOLVED

The purpose of one aspect of the present invention is to provide a pharmaceutical composition for preventing or treating a cancer in which a HER2 expression level is IHC 1+ or higher, comprising a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta as an active component.

The purpose of another aspect of the present invention is to provide a pharmaceutical composition for preventing or treating a cancer in which a HER2 expression level is IHC 1+ or higher, consisting of a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta.

The purpose of another aspect of the present invention is to provide a pharmaceutical composition for preventing or treating a cancer in which a HER2 expression level is IHC 1+ or higher, consisting essentially of a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta.

The purpose of another aspect of the present invention is to provide use of a recombinant protein comprising an interferon-beta or a variant of an interferon-beta of SEQ ID NO:1 in which the 27^{th} amino acid residue is substituted with threonine; and a HER2-tarteting antibody or a fragment thereof covalently linked directly or indirectly to the interferon-beta to prepare a therapeutic agent for a cancer with a HER2 expression level of IHC 1+ or higher.

The purpose of another aspect of the present invention is to provide a method of treating a cancer in which a HER2 expression level is IHC 1+ or higher, comprising administering an effective amount of a composition to a subject in need thereof, the composition protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta or the fragment thereof as an active component.

The purpose of another aspect of the present invention is to provide a pharmaceutical composition for preventing or treating a cancer in which a HER2 expression level is IHC 1+ or higher, comprising an interferon-beta or a variant of an interferon-beta of SEQ ID NO:1 in which the 27^{th} amino acid residue is substituted with threonine; and a HER2-targeting antibody or a fragment thereof as active components.

The purpose of another aspect of the present invention is to provide a pharmaceutical composition for preventing or treating a cancer in which a HER2 expression level is IHC 1+ or higher, consisting of an interferon-beta or a variant of an interferon-beta of SEQ ID NO:1 in which the 27^{th} amino acid residue is substituted with threonine; and a HER2-targeting antibody or a fragment thereof.

The purpose of another aspect of the present invention is to provide a pharmaceutical composition for preventing or treating a cancer in which a HER2 expression level is IHC 1+ or higher, essentially consisting of an interferon-beta or a variant of an interferon-beta of SEQ ID NO:1 in which the 27^{th} amino acid residue is substituted with threonine; and a HER2-targeting antibody or a fragment thereof.

The purpose of another aspect of the present invention is to provide use of a composition comprising an interferon-beta or a variant of an interferon-beta of SEQ ID NO:1 in which the 27^{th} amino acid residue is substituted with threonine; and a HER2-targeting antibody or a fragment thereof to prepare a therapeutic agent for cancer with a HER2 expression level of IHC 1+ or higher.

The purpose of another aspect of the present invention is to provide a method of treating a cancer in which a HER2 expression level is IHC 1+ or higher, comprising administering an effective amount of a composition to a subject in need thereof, the composition comprising an interferon-beta or a variant of an interferon-beta of SEQ ID NO:1 in which the 27^{th} amino acid residue is substituted with threonine; and a HER2-targeting antibody or a fragment thereof.

### SOLUTIONS TO THE PROBLEMS

According to one embodiment of the present invention, the present invention provides a pharmaceutical composition for preventing or treating a cancer in which a HER2 expression level is IHC 1+ or higher, comprising a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta as an active component.

According to another embodiment of the present invention, the present invention provides a pharmaceutical composition for preventing or treating a cancer in which a HER2 expression level is IHC 1+ or higher, consisting of a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta.

According to another embodiment of the present invention, the present invention provides a pharmaceutical composition for preventing or treating a cancer in which a HER2 expression level is IHC 1+ or higher, consisting essentially of a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta.

According to another embodiment of the present invention, the present invention provides use of a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta to prepare a therapeutic agent for a cancer with a HER2 expression level of IHC 1+ or higher.

According to another embodiment of the present invention, the present invention provides a method of treating a cancer in which a HER2 expression level is IHC 1+ or higher, comprising administering an effective amount of a composition to a subject in need thereof, the composition protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta as an active component.

According to another embodiment of the present invention, the present invention is to provide a pharmaceutical composition for preventing or treating a cancer in which a HER2 expression level is IHC 1+ or higher, comprising an interferon-beta or a variant of an interferon-beta of SEQ ID NO:1 in which the 27^{th} amino acid residue is substituted with threonine; and a HER2-targeting antibody or a fragment thereof as active components.

According to another embodiment of the present invention, the present invention is to provide a pharmaceutical composition for preventing or treating a cancer in which a HER2 expression level is IHC 1+ or higher, consisting of an interferon-beta or a variant of an interferon-beta of SEQ ID NO:1 in which the 27^{th} amino acid residue is substituted with threonine; and a HER2-targeting antibody or a fragment thereof.

According to another embodiment of the present invention, the present invention is to provide a pharmaceutical composition for preventing or treating a cancer in which a HER2 expression level is IHC 1+ or higher, essentially consisting of an interferon-beta or a variant of an interferon-beta of SEQ ID NO:1 in which the 27^{th} amino acid residue is substituted with threonine; and a HER2-targeting antibody or a fragment thereof.

According to another embodiment of the present invention, the present invention is to provide use of a composition comprising an interferon-beta or a variant of an interferon-beta of SEQ ID NO:1 in which the 27^{th} amino acid residue is substituted with threonine; and a HER2-targeting antibody or a fragment thereof to prepare a therapeutic agent for cancer with a HER2 expression level of IHC 1+ or higher.

According to another embodiment of the present invention, the present invention provides a method of treating a cancer in which a HER2 expression level is IHC 1+ or higher, comprising administering an effective amount of a composition to the subject in need thereof, the composition comprising an interferon-beta or a variant of an interferon-beta of SEQ ID NO:1 in which the 27^{th} amino acid residue is substituted with threonine; and a HER2-targeting antibody or a fragment thereof.

Hereinafter, the present invention will be described in detail.

The present invention provides a pharmaceutical composition comprising a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta as an active component for preventing or treating a cancer in which the HER2 expression level is IHC 1+ or higher.

Interferon-beta (IFN-β or IFNβ) is a globular protein with 5 alpha helices, 22 kDa in size, and is reported to be effective in treating cancers, autoimmune disorders, viral infections, HIV-related diseases, hepatitis C, and rheumatoid arthritis due to its diverse immunological activities such as tt activity, cell growth-inhibiting or anti-proliferative activity, lymphocyte cytotoxicity-enhancing activity, immunomodulatory activity, target cell differentiation-inducing or repressing activity, macrophageactivating activity, cytokine production-increasing activity, cytotoxic T cell effect-boosting activity, natural killing cell-increasing activity etc. However, interferon-beta is a strong hydrophobic protein with aggregating property, and has a low biological activity and productivity as well as a short half-life, which limits the utilization of interferon-beta. Thus, the present invention presents a novel development of a recombinant protein conjugated with a variant of interferon-beta showing enhanced expression levels in the cell compared with wild-type interferon-beta with no alterations, by inducing site-directed mutagenesis in the genes of interferon-beta or a recombinant protein fused to wild-type interferon-beta.

A variant of interferon-beta or a mutant of interferon-beta (also referred to as an interferon-beta mutein in the specification) is characterized in that the 27^{th} arginine (R) is substituted with threonine (T), thereby comprising a glycosyl group at the 80^{th} and 25^{th} amino acid residues. In this case, the interferon-beta variant may be one represented by the amino acid sequence of SEQ ID NO:1 or the nucleotide sequence of SEQ ID NO:2 encoding thereof. Such variants of interferon-beta may include all or part of the amino acid sequence of wild-type interferon-beta, and have interferon-beta activity.

The antibody includes monoclonal antibodies, polyclonal antibodies, multi-specific antibodies (e.g., bispecific antibodies) and fragments thereof. A complete antibody is overall Y-shaped and consists of two long heavy chains (H) and two short light chains (L). Each heavy chain and light chain are linked to each other by a sulfide bond, and are divided into a variable region (V), a region that reacts with an antigen, and a constant region (C), a region that expresses an effector function. In the variable region, there is a complementarity-determining region (CDR) that determines the structure of the variable region so as to form specific binding with an antigen and controls antibody binding strength. A fragment of an antibody represents a specific site capable of reacting with an antigen to exhibit antigen-binding activity, for example, a Fab fragment (a fragment by papain digestion), a Fab' fragment (a fragment by pepsin digestion and partial reduction), F (ab')2 fragment (fragment by pepsin digestion), Facb (fragment by plasmin digestion), Fd (fragment by pepsin digestion, partial reduction and re-aggregation), scFv fragment (fragment by molecular biology techniques), etc. Preferably, the antibody is an antibody or fragment thereof that recognizes a tumor-specific antigen, and may be used as a targeted therapy for cancer. As an example, it may be trastuzumab or pertuzumab, which are antibody therapeutics targeting human epidermal growth factor receptor 2 (HER2). In this case, trastuzumab may be composed of a heavy chain having the amino acid sequence indicated by SEQ ID NO:3 and a light chain having the amino acid sequence represented by SEQ ID NO:4.

The recombinant protein may be a fusion or complex protein formed by connecting an interferon-beta or a variant or mutant of the interferon-beta; and an antibody or a fragment thereof by a peptide linker. The peptide linker refers to a short fragment molecule made of two or more amino acids or amino acid analogs linked by peptide bonds to each other, serving to connect two or more separate substances to each other. In this case, linkers such as a glycine-serine linker, a glycine-serine-alanine linker, etc. may be utilized by having glycine, serine, alanine, etc. as major constituent amino acids. Such a linker may be connected to the C-terminus of the antibody heavy chain or the C-terminus of the antibody light chain. The N-terminus of the linker may be connected to the C-terminus of the light chain and the C-terminus of the heavy chain of the antibody. In this case, the C-terminus of the linker may be linked with the N-terminus of the interferon-beta variant. The recombinant protein may be a protein represented by SEQ ID NO:5 having the amino acid sequence in which the interferon-beta variant and the heavy chain of trastuzumab are linked.

Interferon-beta has the strongest effect of inhibiting the growth of cancer cells among various cytokines. However, the treatment using interferon-beta alone acts on the whole body of cancer patients and causes various side effects. Moreover, it is known that symptoms such as fever (80%), muscle pain (73%), headache (50%), fatigue (50%) and malaise (50%) appear in the treated patients.

Human epidermal growth factor receptor 2 (HER2) is a type of tyrosine kinase receptor overexpressed in breast cancer and gastric cancer having a size of 185 kDa, and plays a role in promoting cell growth by activating various intracellular signaling systems. Therefore, various HER2-targeting antibody therapeutics that specifically inhibit HER2 activity and exhibit anti-cancer effects through antibody-dependent cellular cytotoxicity (ADCC) have been developed and are currently being used in patients. However, since the HER2 antibody treatment alone is not effective enough, pertuzumab, another antibody treatment targeting HER2, and chemotherapeutics are being administered in combination. Also, among HER2-positive cancer patients, antibody therapeutics are used only in certain patients with high HER2 expression levels, and still there are other limitations such as cancer recurrence or development of resistance to the current HER2 therapeutics after administration of the therapeutic agent. In particular, pSTAT3, one of the signaling substances involved in the treatment resistance mechanism of trastuzumab, has a form in which two identical substances are polymerized (pSTAT3 homodimer), and is known to be inhibited by pSTAT1. Therefore, increased anti-cancer efficacy can be expected when interferon-beta, which activates pSTAT1, is combined with trastuzumab.

On the other hand, HER2-targeting antibody therapeutics, for example, trastuzumab, are generally prescribed for the treatment of cancer cells in which the HER2 receptor is overexpressed. Cancer cells with HER2 receptor overexpression contain significantly higher levels of the HER2 receptor protein or its gene when compared to noncancerous cells of the same tissue type. Such overexpression may be caused by gene amplification or by increased transcription or translation. HER2 receptor overexpression or amplification can be determined in a diagnostic or prognostic assay by assessing the extent of increase in levels of HER2 protein present on the surface of cells (e.g., via immunohistochemical assays; IHC). Alternatively, or additionally, it can be determined by measuring the level of HER2-encoding nucleic acid in the cells by southern blotting or polymerase chain reaction (PCR) techniques, for example, quantitative real-time PCR (qRT-PCR) or *in situ* hybridization (ISH) such as fluorescence *in situ* hybridization (FISH) and chromophore *in situ* hybridization (CISH).

In general, HER2-targeting antibody therapeutics, for example, trastuzumab, are prescribe to patients having HER2-overexpressing cancer cells with a score of IHC 3+, or to those with the HER2 expression level of IHC 2+ and HER2 gene amplification when analyzed by fluorescent *in situ* hybridization (FISH), thereby being determined as FISH positive (FISH(+)) by a person skilled in the art.

The composition provided by the present invention is characterized in that it can be administered to, and exerts a therapeutic effect in a patient group that normally cannot be prescribed with the conventional HER2-targeting antibody treatment or cannot be expected of any significant treatment efficacy even if prescribed.

In one embodiment of the present invention, the composition of the present invention may be characterized in that it is for preventing or treating cancers with the HER2 expression level of IHC 1+ or higher.

In a preferred embodiment of the present invention, the composition of the present invention may be characterized in that it is for preventing or treating cancers with the HER2 expression level of IHC 1+, or IHC 2+ in combination with negative FISH.

In a preferred embodiment of the present invention, the composition of the present invention may be characterized in that it is for preventing or treating breast cancer or gastric cancer with the HER2 expression level of IHC 1+ or higher.

In a preferred embodiment of the present invention, the composition of the present invention may be characterized in that it is for preventing or treating breast cancer or gastric cancer with the HER2 expression level of IHC 1+, or IHC 2+ in combination with negative FISH.

The pharmaceutical composition of the present invention may be formulated in various ways together with a pharmaceutically acceptable carrier according to the route of administration by a method known in the art. "Pharmaceutically acceptable" refers to a non-toxic composition that is physiologically acceptable and does not inhibit the action of the active ingredient and does not normally cause allergic reactions such as gastrointestinal disorders, dizziness, or similar reactions when administered to humans. The carrier includes all kinds of solvents, dispersion media, oil-in-water or water-in-oil emulsions, aqueous compositions, liposomes, microbeads and microsomes.

The route of administration may be oral or parenteral administration. Parenteral administration methods include, but not limited to, intravenous, intramuscular, intra-arterial, intramedullary, intrathecal, intra-cardiac, transdermal, subcutaneous, intraperitoneal, intranasal, enteral, topical, sublingual or rectal administration.

When the pharmaceutical composition of the present invention is orally administered, the pharmaceutical composition of the present invention may be formulated in forms such as powder, granule, tablet, pill, dragee, capsule, liquid, or gel, syrup, suspension, wafer, and the like together with a suitable carrier for oral administration according to methods known in the art. Examples of suitable carriers may include sugars such as lactose, dextrose, sucrose, sorbitol, mannitol, xylitol, erythritol and maltitol, and starches including corn starch, wheat starch, rice starch and potato starch, and celluloses including cellulose, methyl cellulose, sodium carboxymethylcellulose and hydroxypropylmethylcellulose and the like, and fillers such as gelatin, polyvinylpyrrolidone, and the like. In addition, cross-linked polyvinylpyrrolidone, agar, alginic acid or sodium alginate may be added as a disintegrant if necessary. Furthermore, the pharmaceutical composition may further include an anti-aggregating agent, a lubricant, a wetting agent, a flavoring agent, an emulsifying agent, and a preservative.

In addition, when administered parenterally, the pharmaceutical composition of the present invention may be formulated according to methods known in the art in the form of injections, transdermal administrations, and nasal inhalants together with suitable parenteral carriers. In the case of the injection, it must be sterilized and protected from contamination by microorganisms such as bacteria and fungi. For injection, examples of suitable carriers include, but not limited to, water, ethanol, polyols (e.g., glycerol, propylene glycol and liquid polyethylene glycol, etc.), mixtures thereof, and/or a solvent or dispersion medium containing vegetable oil. More preferably, suitable carriers may be selected among Hanks' solution, Ringer' s solution, phosphate buffered saline (PBS) containing triethanolamine, or isotonic solutions such as sterile water for injection, 10% ethanol, 40% propylene glycol and 5% dextrose. In order to protect the injection from microbial contamination, it may further include various antibacterial and antifungal agents such as parabens, chlorobutanol, phenol, sorbic acid, thimerosal. In addition, in most cases, the injection may further contain an isotonic agent such as sugar or sodium chloride. In the case of transdermal administration, forms such as ointments, creams, lotions, gels, external solutions, pasta agents, liniments, and air rolls are included. As used herein, "transdermal administration" means that an effective amount of the active ingredients contained in the pharmaceutical composition is delivered into the skin by topically administering the pharmaceutical composition to the skin. For example, the pharmaceutical composition of the present invention may be prepared as an injectable formulation and be administered by lightly pricking the skin with a 30-gauge thin injection needle or directly applying to the skin. These formulations are described in formulary commonly known in pharmaceutical chemistry.

In the case of administration by inhalation, the compounds used according to the invention may be conveniently delivered in the form of aerosol spray from the pressurized packs or a nebulizer using a suitable propellant, for example, dichlorofluoromethane, trichlorofluoromethane, dichlorotetrafluoroethane, carbon dioxide or other applicable gas. In the case of pressurized aerosols, the dosage unit may be determined by providing a valve to deliver a metered amount. For example, gelatin capsules and cartridges used in inhalers or insufflators may be formulated to contain a powder mixture of the compound and a suitable powder base such as lactose or starch. As for other pharmaceutically acceptable carriers, those known in the art may be referred to.

In addition, the pharmaceutical composition according to the present invention may contain one or more buffers (e.g., saline or PBS), carbohydrates (e.g., glucose, mannose, sucrose or dextran), antioxidants, bacteriostatic agents, chelating agents (e.g., EDTA or glutathione), adjuvants (e.g., aluminum hydroxide), suspending agents, thickening agents and/or preservatives.

In addition, the pharmaceutical compositions of the present invention may be formulated using methods known in the art to provide rapid, sustained or delayed release of the active ingredient after administration to a mammal.

In addition, according to another embodiment of the present invention, a method of treating a cancer in which the HER2 expression level is IHC 1+ or higher comprising the following steps is provided:
a) obtaining a cancer tissue from a patient;
b) performing an immunohistochemical staining with the obtained cancer tissue;
c) selecting a HER2-positive cancer patient with IHC 1+ or higher in step b); and
d) administering a recombinant protein comprising an interferon-beta or a variant of interferon-beta of SEQ ID NO:1 in which the 27^{th} amino acid
residue is substituted with threonine; and a HER2-targeting antibody or a fragment thereof covalently linked directly or indirectly to the interferon-beta or the variant thereof to the HER2-positive cancer patient with IHC 1+ or higher.

HER2 antibody therapeutics currently on the market, namely trastuzumab and pertuzumab, are being used to treat patients with HER2-positive breast cancer or gastric cancer. They are used only for the patients with very high levels of HER2 expression (IHC 3+), or moderate levels of expression (IHC 2+) with positive FISH results when immunohistochemistry (IHC) is performed on cancer tissues of HER2 patients. This is because these antibody therapeutics are ineffective in patients with low HER2 expression levels.

The fusion protein of the HER2 antibody and interferon-beta or its variant provided in the present invention shows further enhanced anti-cancer activity because effects of cancer treatment and immune activation from interferon-beta or its variant are added, while inhibition of HER2 signal transduction and ADCC which is the anti-cancer function of the existing antibody therapeutics are kept mainly unaltered. Therefore, when selecting HER2-positive cancer patients to administer the fusion protein for the purpose of cancer treatment, it should be based on different standards from those of the current HER2 antibody therapeutics.

In selecting a patient to administer the fusion protein of the HER2-targeting antibody and interferon-beta or its variant, IHC is performed on the cancer tissue from a cancer patient, and screened for the expression level of IHC 1+ or higher.

According to a preferred aspect of the present invention, it may be characterized in that step d) may be performed after selecting a patient with IHC 1+ in step c).

According to a preferred embodiment of the present invention, it may be characterized in that patients with IHC 2+ are selected in step c) and further subjected to the additional FISH analysis. As a result, step d) may be performed with the patient with negative FISH result among IHC 2+ patients.

The present invention provides a use of a recombinant protein comprising an interferon-beta or a variant of an interferon-beta of SEQ ID NO:1 in which the 27^{th} amino acid residue is substituted with threonine; and an HER2-targeting antibody or its fragment covalently linked directly or indirectly to the interferon-beta or its variant.

The present invention provides a method of treating cancer in which a HER2 expression level is IHC 1+ or higher, comprising administering an effective amount of a composition to a subject in need thereof, comprising a recombinant protein comprising an interferon-beta or a variant of an interferon-beta of SEQ ID NO:1 in which the 27^{th} amino acid residue is substituted with threonine; and a HER2-targeting antibody or a fragment thereof covalently linked directly or indirectly to the interferon-beta or the variant thereof as an effective ingredient.

The 'effective amount' of the present invention refers to an amount that, when administered to an individual or a subject, has an effect of improving, treating, detecting, diagnosing, or ameliorating, or suppressing the progress of the cancer with the HER2 expression level of IHC 1+ or higher. The 'subject' may be an animal comprising preferably a mammal, particularly a human, and may be an animal-derived cell, tissue, organ, etc. The subject may be a patient in need of the effect.

The 'treatment' of the present invention comprehensively refers to alleviating cancer-related symptoms or the progress of cancer in which the HER2 expression level of IHC 1+ or higher, and it may include curing, substantially preventing, or improving the condition of the HER2-positive cancer. Further, it also includes, but is not limited to, alleviating, curing, or preventing one symptom or most of the symptoms resulting from the disease.

As used herein, the term "comprising" is used in the same sense as "including" or "characterized by" , and in the composition or method according to the present invention, any additional components or method steps that have not been specifically mentioned or described are not excluded. In addition, the term "consisting of" means excluding additional elements, steps, or ingredients that are not specifically described. The term "essentially consisting of" means that, in the scope of a composition or method, it may include substances or steps that do not substantially affect its basic properties in addition to the described substances or steps.

The present invention provides a pharmaceutical composition for preventing or treating a cancer in which a HER2 expression level is IHC 1+ or higher, comprising an interferon-beta or a variant of interferon-beta of SEQ ID NO:1 in which the 27th amino acid residue is substituted with threonine; and an a HER2-targeting antibody or a fragment thereof as active components.

The pharmaceutical composition according to the present invention acts to augment effects of preventing and treating cancer in which the HER2 expression level of IHC 1+ or higher, by concurrent administration of interferon-beta or an interferon-beta variant of SEQ ID NO: 1 in which the 27^{th} amino acid residue is substituted with threonine; and a HER2-targeting antibody or its fragment.

More specifically, a remarkable synergism may appear in treating cancer by co-administrating an interferon-beta protein or a variant of interferon-beta of SEQ ID NO:1 in which the 27^{th} amino acid residue is substituted with threonine and the HER2-targeting antibody or its fragment to a patient group that does not improve by each drug alone, that is, a cancer patient group with low HER2 expression levels. Such synergistic effect of combined administration is now disclosed for the first time in the present invention.

In the present invention, the term 'synergy' means that, as described in the literature, the effect generated when each component is administered in combination is greater than the sum of the effects generated when administered alone as a single component (Chou and Talalay, Adv. Enzyme. Regul., 22:27-55, 1984).

In the present invention, the term 'administered in combination' means that two or more components are administered together to a subject. When individual components are administered together, it means that each component may be administered sequentially at the same time or in any order or at different times to obtain the desired therapeutic effect.

When interferon-beta or the interferon-beta variant of SEQ ID NO: 1 in which the 27th amino acid residue is substituted with threonine; and the HER2-targeting antibody or its fragment are formulated individually or separately, and administered in combination in the form of separate compositions, the formulations may be prepared according to a known method respectively.

In the meantime, interferon-beta or the interferon-beta variant of SEQ ID NO:1 in which the 27^{th} amino acid residue is substituted with threonine; and the HER2-targeting antibody or its fragment may be formulated in the form of a single composition, and the formulation can be prepared according to a known method.

As for the pharmaceutical composition comprising an interferon-beta or its variant of SEQ ID NO:1 in which the 27^{th} amino acid residue is substituted with threonine; and a HER2-targeting antibody or a fragment thereof according to the present invention, each component may be administered simultaneously, separately or sequentially. For example, when individual components included in the pharmaceutical composition of the present invention are formulated together as a single composition, they can be administered at the same time. When it is not prepared as a single composition, one component may be administered before or after another component and/or together with the other component at the same time. The order of administration of the pharmaceutical composition according to the present invention, that is, whether to administer which, at what point in time, simultaneously, individually or sequentially, can be determined by a doctor or an expert. This order of administration may change depending on various factors.

In one aspect of the present invention, the composition of the present invention may be characterized in that it is for preventing or treating a cancer in which the HER2 expression level is IHC 1+ or higher.

In a preferred embodiment of the present invention, the composition of the present invention may be characterized in that it is for preventing or treating a cancer in which the HER2 expression level is IHC 1+, or IHC 2+ in combination with negative FISH.

In a preferred embodiment of the present invention, the composition of the present invention may be characterized in that it is for preventing or treating breast cancer or gastric cancer in which the HER2 expression level is IHC 1+ or higher.

In a preferred embodiment of the present invention, the composition of the present invention may be characterized in that it is for preventing or treating breast cancer or gastric cancer in which the HER2 expression level is IHC 1+, or IHC 2+ in combination with negative FISH.

The present invention also provides a use of a composition comprising an interferon-beta or a variant of an interferon-beta of SEQ ID NO:1 in which the 27^{th} amino acid residue is substituted with threonine; and a HER2-targeting antibody or a fragment thereof to prepare a therapeutic agent for a cancer with a HER2 expression level of IHC 1+ or higher.

The present invention also provides a method of treating a cancer in which a HER2 expression level is IHC 1+ or higher, comprising administering an effective amount of a composition to a subject in need thereof, comprising an interferon-beta or a variant of an interferon-beta of SEQ ID NO:1 in which the 27^{th} amino acid residue is substituted with threonine; and a HER2-targeting antibody or a fragment thereof.

### EFFECTS OF THE INVENTION

The recombinant protein according to the present invention in which interferon-beta or its variant and a HER2-targeting antibody or its fragment are fused to each other can be used for the purpose of treating patients with HER2-positive cancer. In addition, the present invention provides a method for selecting HER2-positive cancer patients to whom the recombinant protein will be administered such that a greater number of patients can benefit from the treatment than the conventional HER2 antibody therapeutics.

### BRIEF EXPLANATIONS OF THE DRAWINGS

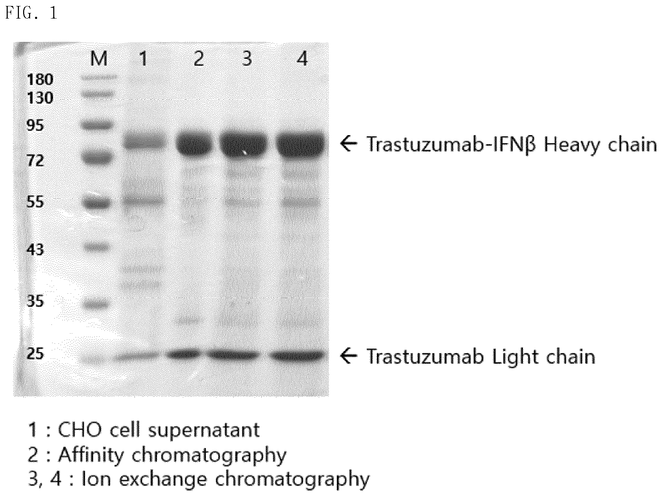

Figure 1 shows results from trastuzumab-IFNβ mutein purification experiments. SDS-PAGE was performed with proteins isolated from the first step of affinity chromatography using protein A beads, and proteins subsequently separated by ion exchange chromatography.

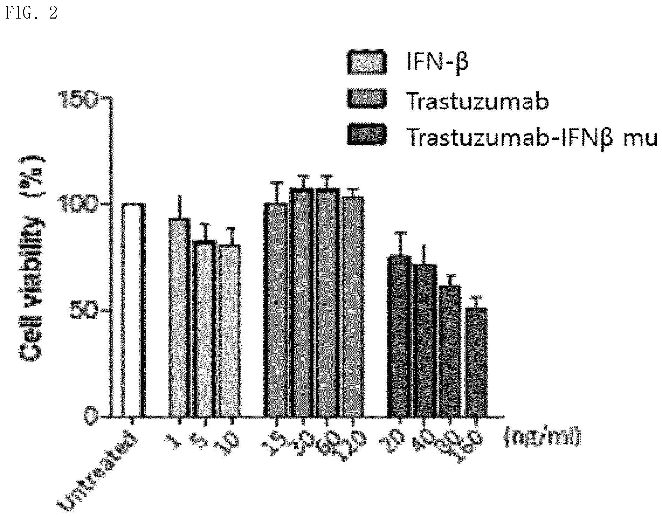

Figure 2 is a graph showing direct cellular toxicity of trastuzumab and trastuzumab-IFNβ mutein. NCI-N87 cells were grown in a 96-well plate, and received treatments of trastuzumab or trastuzumab-IFNβ mutein at different concentrations. After 72 hours, WST assays were performed to evaluate percentages of living cells. Although cytotoxicity was not observed in the trastuzumab-treated group, it was confirmed that the cytotoxic effect appeared only in the trastuzumab-IFNβ-treated group.

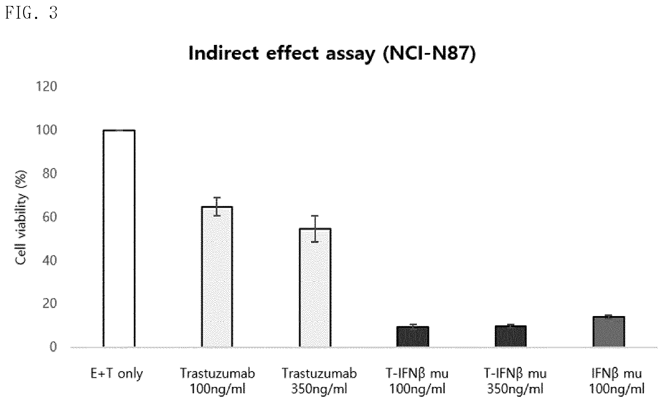

Figure 3 is a graph showing cytotoxicity results from the indirect immune activation of trastuzumab or trastuzumab-IFNβ mutein. NCI-N87 cells were cultured in a 96-well plate, then treated with trastuzumab or trastuzumab-IFNβ mutein at different concentrations along with PBMCs. 48 hours later, percentages of viable cells were measured by WST assay.

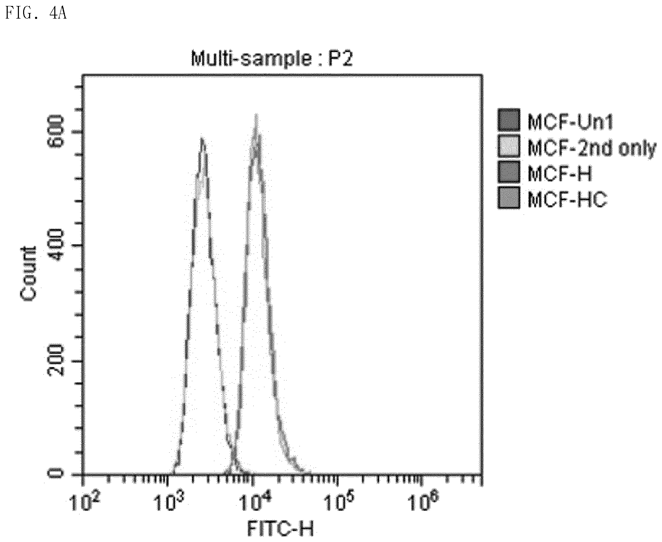

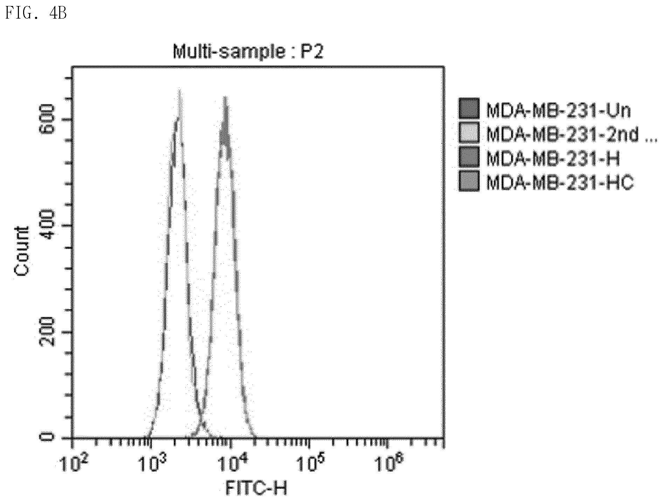

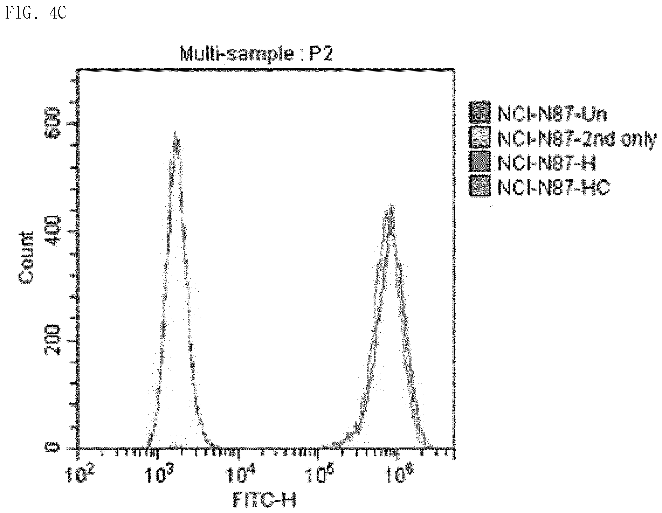

Figures 4a to 4c show flow cytometry results to analyze the HER2-binding ability of trastuzumab and trastuzumab-IFNβ mutein. Three different cell lines including MCF-7 (FIG.4a), MDA-MB-231 (FIG.4b), and NCI-N87 (FIG.4c) were reacted with trastuzumab or trastuzumab-IFNβ mutein and measured for their binding ability to HER2 by flow cytometry.

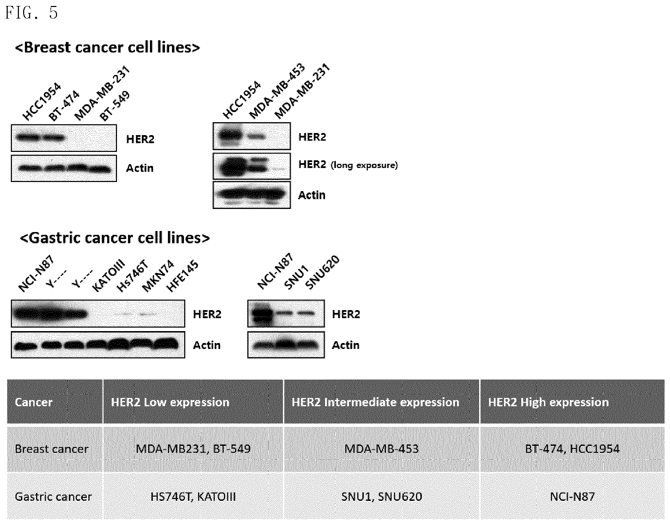

Figure 5 is western blotting results to compare HER2 expression levels in breast cancer cell lines and gastric cancer cell lines. Based on the HER2 expression level of each cell line, they were classified as HER2 low expression, HER2 intermediate expression, and HER2 high expression.

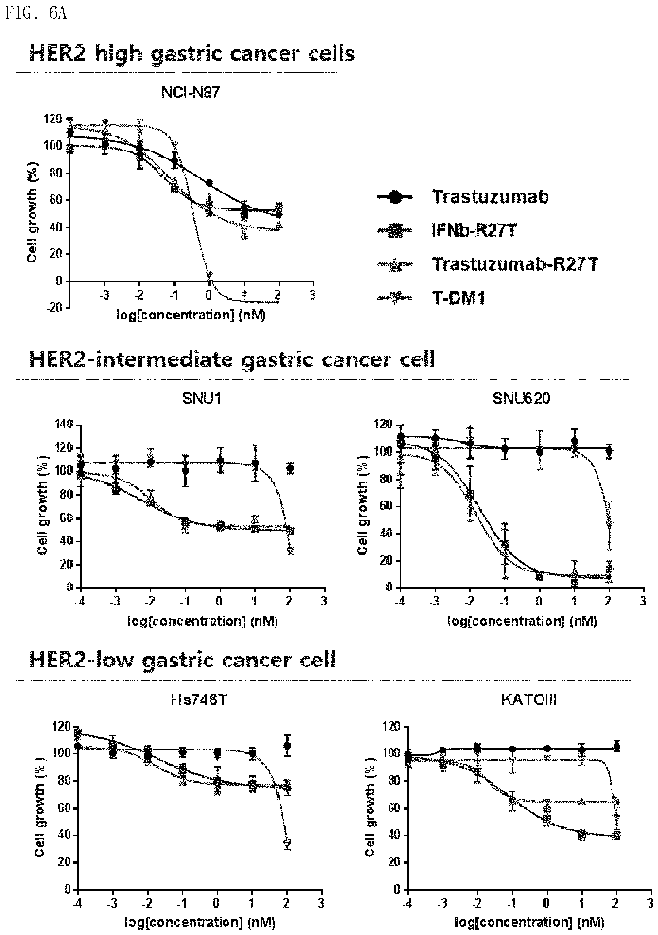

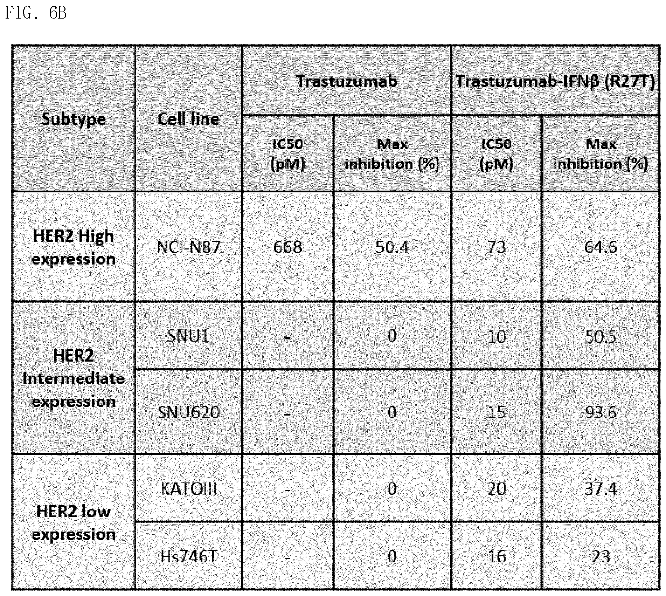

Figures 6a and 6b show direct cytotoxicity results of trastuzumab and trastuzumab-IFNβ mutein in gastric cancer cell lines. Gastric cancer cell lines with different HER2 expression levels were cultured in 96-well plates, and subjected to trastuzumab or trastuzumab-IFNβ mutein treatment at different concentrations. 72 hours later, percentages of viable cells were confirmed by WST assay.

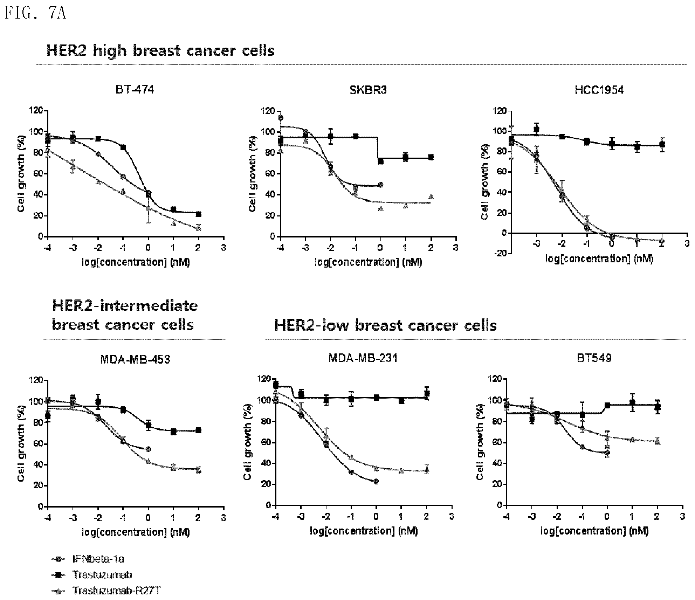

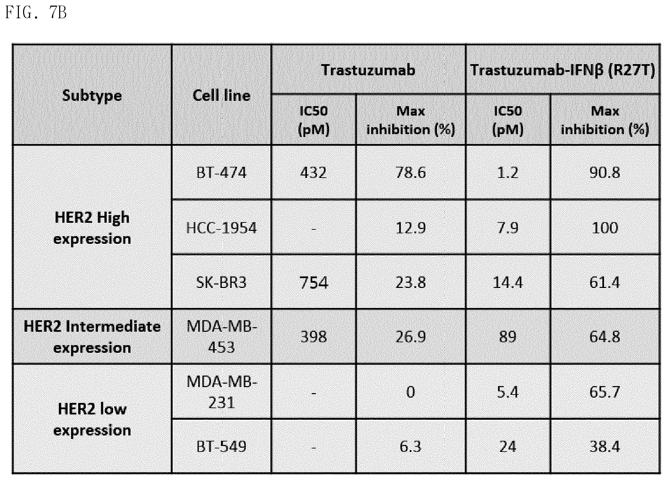

Figures 7a and 7b show direct cytotoxicity results of trastuzumab and trastuzumab-IFNβ mutein in breast cancer cell lines. Breast cancer cell lines with different HER2 expression levels were cultured in 96-well plates, and then treated with different concentrations of trastuzumab or trastuzumab-IFNβ mutein. Following 72 hours, percentages of viable cells were confirmed by WST assay.

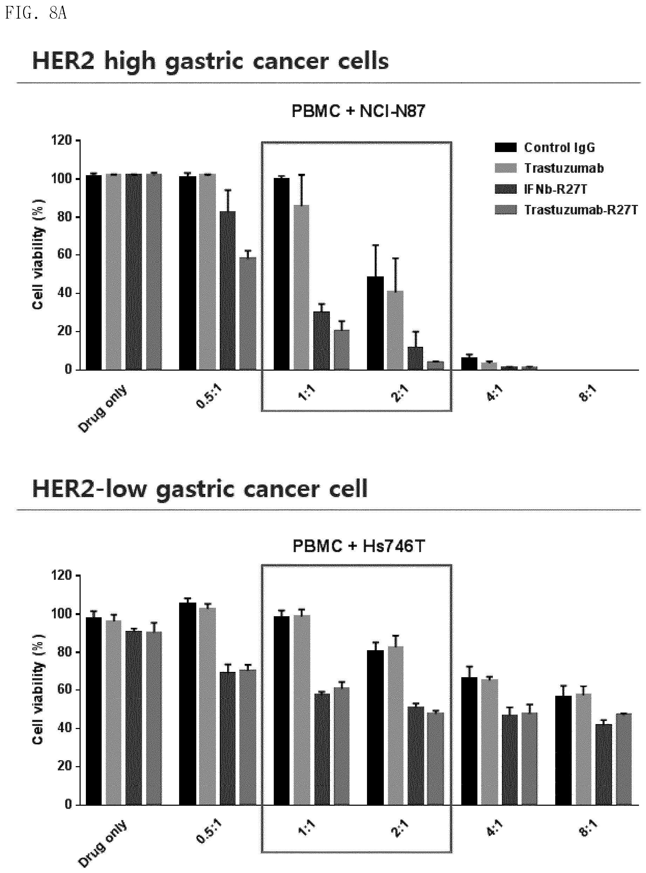

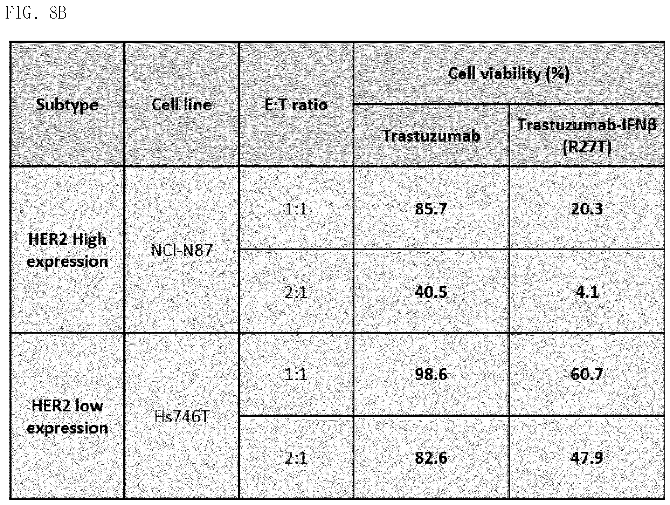

Figures 8a and 8b are cytotoxicity results of indirect immune activation by trastuzumab and trastuzumab-IFNβ mutein in gastric cancer cell lines. Cell lines with different HER2 expression levels were cultured in 96-well plates, and were treated with different concentrations of trastuzumab or trastuzumab-IFNβ mutein together with PBMCs. 48 hours later, percentages of living cells were confirmed by WST assay.

### DETAILED EXPLANATION OF THE INVENTION

Hereinafter, the present invention will be described in more detail with reference to the accompanying drawings. However, these descriptions are provided for illustrative purposes only to help the understanding of the present invention, and the scope of the present invention is not limited by these illustrative descriptions.

### 1. Materials and methods

### 1-1. Design to prepare fusion proteins.

In order to establish stable cells and cell lines transiently expressing the antibody fused to the mutant protein, pCHO 1.0 (Life Technologies) was used as an expression vector. Trastuzumab was used as an antibody, and the variant of interferon-beta in which the 27th amino acid residue was substituted with threonine was used as a mutant protein (mutein).

The interferon-beta variant was fused to the heavy chain region of the antibody. A linker was cloned into the heavy chain region of the antibody, and the interferon-beta variant was cloned therein, respectively. Thereafter, a restriction enzyme AvrII cleavage site (CCTAGG) and a Bstz17I cleavage site (GTATAC) (enzymes from Thermo Scientific, USA) were inserted into the 3' and 5' -ends of the entire gene to secure the final gene of the heavy chain. In addition, restriction enzyme EcoRV site (GATATC) and PacI site (TTAATTAA) were inserted into the 3' and 5' -ends of the light chain of the antibody to secure the final gene of the light chain. The final genes of the heavy and light chains were inserted into the pCHO 1.0 vector.

### 1-2. Expression of fusion protein constructs in mammalian cells

Expression vectors of trastuzumab and the trastuzumab-fused interferon-beta variant (Trastuzumab-IFNβ mutein) were transformed into CHO-S cells (Thermo Scientific) using FreeStyle^{™} MAX reagent (Thermo Scientific). A mixture obtained by adding OptiPRO^{™} SFM (Thermo Scientific) to the FreeStyle^{™} MAX reagent-DNA complex was put into the CHO-S cells in a flask, and incubated in a humidified 8% CO₂ atmosphere at atmospheric pressure. Stable transformants to express the fusion protein were selected 48 hours after transformation. Cells were sorted through secondary selection with puromycin 10-50 µg/mL and MTX 100~1,000nM. Selected cells were incubated in the presence of glucose to express the fusion protein for 14 days at 37°C under humidified 8% CO₂ atmospheric pressure and 130 rpm.

### 1-3. Purification of fusion proteins

Fusion proteins expressed in CHO-S cells were isolated by affinity chromatography and ion exchange chromatography. After passing the CHO-S culture medium through a column filled with protein A Mabselect sure (GE Healthcare), equilibration buffer, wash buffer, and elution buffer were sequentially eluted to obtain purified proteins. In case of the ion exchange chromatography purification method, fusion proteins obtained by affinity chromatography purification were passed through a column filled with HiTrap Q (HiTrap Q FF, GE healthcare), then elution buffer was added to obtain purified proteins.

### 1-4. Cell lines and culture conditions

The human gastric carcinoma (NCI-N87) cell line and the human breast cancer (MCF-7, MDA-MB-231) cell line were purchased from the Korean Cell Line Bank (KCLB).

NCI-N87 cells were cultured in RPMI-1640 (HyClone, USA) culture medium containing 10% FBS (HyClone), penicillin 100 units/mL and streptomycin 100 µg/mL, while MCF-7 and MDA-MB231 cells were cultured using DMEM (HyClone, USA) culture medium containing 10% FBS (HyClone), penicillin 100 units/mL, and streptomycin 1001µg/mL at 37°C under atmospheric pressure of 5% CO₂ with humidity.

### 1-5. Examination of direct cytotoxicity of interferon-beta

In a 96-well plate, 2.0×10⁴ (200µl) NCI-N87 cells were incubated in each well and divided into a control group and treatment groups of IFNβ, trastuzumab, or trastuzumab-IFNβ mutein. On the following day, IFNβ (1, 5, 10 ng/ml), trastuzumab (15, 30, 60, 120 ng/ml), or trastuzumab-IFNβ mutein (20, 40, 80, 160 ng/ml) were administered at the corresponding concentrations, respectively, and incubated for 72 hours. Then, 10µl of EZ-Cytox (Dae-il Biotech) was added to each well, and reacted for 3 hours in an incubator. Absorbance at 450 nm was measured and compared using a SpectraMax iD3 multi-mode microplate reader (Molecular Device).

### 1-6. Examination of indirect cytotoxicity of interferon-beta

In a 96-well plate, 2.0×10⁴ (200µl) NCI-N87 cells were incubated in each well and divided into a control group and treatment groups of IFNβ , trastuzumab, or trastuzumab-IFNβ mutein. In the following day, peripheral blood mononuclear cells (PBMC) (Zenbio) were added at a ratio of 20:1 to cancer cells, and IFNβ (100 ng/ml), trastuzumab (100, 350 ng/ml) or trastuzumab-IFNβ mutein (100, 350 ng/ml) was treated to the corresponding concentration, respectively, and cultured for 48 hours. Then, 10µl of EZ-Cytox (Daeil Biotech) was added to each well, and the reaction was carried out in an incubator for 3 hours. Absorbance at 450 nm was measured and compared using a SpectraMax iD3 multi-mode microplate reader (Molecular Device).

### 1-7. Flow cytometry

To measure the antibody' s binding ability to HER2, flow cytometry analysis was performed. NCI-N87, MDA-MB-231, and MCF-7 cells were recovered using cell dissociation buffer (Enzyme-Free, PBS-based) (Gibco) and their cellular activities were inhibited in cold PBS (containing 2% FBS) for 1 hour at 4°C. Cells were then washed with PBS three times and incubated with 1µg of trastuzumab and trastuzumab-IFNβ mutein diluted in PBS for 30 minutes at 4°C. Subsequently cells were washed three times with PBS and then incubated with goat anti-Human IgG FITC (Jackson) at 4°C for 30 minutes. Fluorescent antibodies were measured by flow cytometry (CytoFLEX Flow Cytometer) (Beckman Coulter).

### 1-8. Analysis of endogenous HER2 expression in cancer cells

To measure HER2 expression levels in breast cancer cell lines (HCC1954, BT-474, MDA-MB-231, BT-549) and gastric cancer cell lines (NCI-N87, KATOIII, Hs746T, MKN74, HFE145, SNU1, SNU620), western blot experiments were performed.

Each cell lines were cultured for 7 days and the culture medium was collected and centrifuged to remove cells (8000 rpm, 10 minutes). A small amount of the cell-removed culture medium was collected, mixed with 5× sample buffer, and boiled for 10 minutes at 100°C to induce sufficient protein denaturation. Subsequently prepared protein samples were loaded on a tricine SDS-PAGE gel with a marker, and electrophoresis was performed at a voltage of 130v for 1 hour and 30 minutes. Then, the gel was separated and placed on a 3M paper with a PVDF membrane placed on top of the gel, and another 3M papers were layered, immersed in 1× transfer buffer, and proteins were transferred at the voltage of 100v for 70 minutes. Tris-buffered saline-Tween 20 (TBS-T, 0.1% Tween 20) was added with 5% and the membrane was blocked at room temperature for 1 hour and 30 minutes. The protein-transferred PVDF membrane was washed twice with TBS-T, and left immersed in TBS-T. Anti-HER2 antibody was prepared by diluting in TBS-T at the ratio of 1:1000. The membrane was then immersed in the antibody-diluted solution, reacted at room temperature for 2 hours. After completing this process, the membrane was washed with TBS-T 3 times for 10 minutes. Secondary antibodies conjugated with horseradish peroxidase (HRP) were added and reacted for 1 hour at room temperature. After washing one more time, protein bands were confirmed with ECL reagent (enhanced chemiluminescence reagent, Intron). The band intensity was measured using C-DiGit (LI-COR, USA).

### 1-9. Examination of direct cytotoxicity in the gastric cancer cell lines

Experiments were performed in the same manner as in the above 1-5.

In a 96-well plate, each of NCI-N87, SNU1, SNU620, Hs746T, and KATOIII cells were cultured at the density of 2.0×10⁴ cells (200µl) per well, and within each cell lines, treatment groups were assigned such as IFNβ-R27T treatment group, trastuzumab treatment group, trastuzumab-IFNβ mutein (trastuzumab- R27T) treatment group and T-DM1 treatment group. On the following day, IFNβ (1, 5, 10 ng/ml), trastuzumab (15, 30, 60, 120 ng/ml), or trastuzumab-IFNβ mutein (20, 40, 80, 160 ng/ml) were administered at the corresponding concentrations, respectively, and incubated for 72 hours. Then, 10µl of EZ-Cytox (Daeil Biotech) was added to each well, and the reaction was carried out in an incubator for 3 hours. Absorbance at 450 nm was measured and compared using a SpectraMax iD3 multi-mode microplate reader (Molecular Device).

### 1-10. Examination of direct cytotoxicity in the breast cancer cell lines

Experiments were performed in the same manner as in the above 1-5.

In a 96-well plate, each of BT-474 cells, SKBR3 cells, HCC1954 cells, MDA-MB-453 cells, MDA-MB-231 cells, and BT549 cells were cultured at the density of 2.0×10⁴ (200µl) per well, and for each cell lines, treatment groups such as IFNβ treatment, trastuzumab treatment, and trastuzumab-IFNβ mutein (trastuzumab-R27T) treatment were assigned. On the following day, IFNβ (1, 5, 10 ng/ml), trastuzumab (15, 30, 60, 120 ng/ml), or trastuzumab-IFNβ mutein (20, 40, 80, 160 ng/ml) were administered at the corresponding concentrations, respectively and incubated for 72 hours. Then, 10µl of EZ-Cytox (Daeil Biotech) was added to each well, and the reaction was carried out in an incubator for 3 hours. Absorbance at 450 nm was measured and compared using a SpectraMax iD3 multi-mode microplate reader (Molecular Device).

### 1-11. Examination of indirect cytotoxicity in the gastric cancer cell lines

In a 96-well plate, each of N87 cells, KATOIII cells, Hs746T cells or MKN74 cells were divided into treatment groups of IFNβ, trastuzumab, or trastuzumab-IFNβ mutein (trastuzumab-R27T) and cultured at the concentration of 1.0×10⁴ cells (200µl) per well. The next day, peripheral blood mononuclear cells (PBMC) (Zenbio) were added at a ratio of 1:1 or 1:2 to cancer cells, and IFNβ (100 ng/ml), trastuzumab (100, 350 ng/ml), or trastuzumab-IFNβ mutein (100, 350 ng/ml) were treated at the corresponding concentrations, respectively, and cultured for 3 days. Then, 10µl of EZ-Cytox (Daeil Biotech) was added to each well, and the reaction was carried out in an incubator for 3 hours. Absorbance at 450 nm was measured and compared using a SpectraMax iD3 multi-mode microplate reader (Molecular Device).

### 2. Results

### 2-1. Expression and purification of trastuzumab-IFNβ mutein fusion protein

Protein expression and purification experiments were performed under the same conditions using cell lines expressing trastuzumab-IFNβ mutein and trastuzumab. For expression conditions, CHO-S cell line was cultured at 37°C and 5% CO₂ for 10 days. Concentrations of the expressed fusion protein present in the cell culture media were measured using Cedex-bio (Roche), and purified by affinity chromatography employing AKTA instrument system and protein A beads. Thereafter, secondary purification was performed using ion exchange chromatography.

As shown in FIG.1, it was confirmed that both the heavy chain and light chain proteins of trastuzumab-IFNβ mutein, which were produced through the two-step purification process, were observed at the position corresponding to the expected sizes.

### 2-2. Anti-cancer efficacy of trastuzumab-IFNβ mutein fusion protein

WST assay was used to analyze a direct cytotoxic effect of the fusion protein and indirect anti-cancer efficacy through immune activation.

Referring to FIG.2, trastuzumab-IFNβ mutein exhibited direct cytotoxicity against NCI-N87 cells, however, trastuzumab was not associated with cellular toxicity. This suggests that blocking of the signal transduction by trastuzumab does not cause significant damages or toxicity in the cells.

Referring to FIG.3, trastuzumab-IFNβ mutein showed cytotoxicity in NCI-N87 cells through immune cell activation, and showed even greater cytotoxicity against cancer cells compared to trastuzumab.

### 2-3. Targeting HER2 by trastuzumab-IFNβ mutein

To examine the binding ability to HER2, flow cytometry analysis was performed.

Referring to FIG.4, it was confirmed that trastuzumab and trastuzumab-IFNβ mutein had the same HER2 binding ability in the cell line with a high HER2 expression level (NCI-N87). Similarly, it was observed that trastuzumab and trastuzumab-IFN β mutein had the comparable HER2 binding ability even in cell lines with low HER2 expression levels (MCF-7, MDA-MB-231). These results showed that there was no change in the HER2-binding ability of trastuzumab even when it is fused to IFNβ mutein.

### 2-4. Analysis of endogenous HER2 expression levels in cancer cell lines

Western blot experiments were carried out to compare HER2 expression levels in breast cancer cell lines and gastric cancer cell lines. The HER2 expression levels of breast cancer cell lines HCC1954, BT-474, MDA-MB-231, BT-549 and gastric cancer cell lines NCI-N87, KATOIII, Hs746T, MKN74, HFE145, SNU1, and SNU620 were compared and classified into three groups.

As shown in FIG. 5, HER2 low expression group include MDA-MB231 cells and BT-549 cells among breast cancer cell lines, and HS746T cells and KATOIII cells among gastric cancer cell lines. HER2 intermediate expression group include MDA-MB-453 breast cancer cells, and SNU1 and SNU620 gastric cancer cells. BT-474 and HCC1954 cells among breast cancer cell lines, and NCI-N87 cells among gastric cancer cells fall into HER2 high expression group.

### 2-5. Direct cytotoxic efficacy depending on HER2 expression levels in breast cancer cell lines and gastric cancer cell lines

Direct cytotoxic efficacy of the fusion protein was measured by WST assay method in NCI-N87 cells showing high levels of HER2 expression, SNU1 and SNU620 cells with medium levels of HER2 expression, and Hs746T and KATOIII cells with low levels of HER2 expression.

As shown in FIGS.6a and 6b, trastuzumab-IFNβ mutein showed direct cytotoxic efficacy against cells with medium HER2 expression levels and gastric cancer cells with low expression levels, while trastuzumab did not show any cytotoxic effect.

In addition, as shown in FIGS.7a and 7b, trastuzumab-IFNβ mutein showed direct cytotoxic efficacy against cells with medium HER2 expression levels and breast cancer cells with low expression levels.

### 2-6. Indirect cytotoxic effect depending on HER2 expression level in gastric cancer cell line

NCI-N87 cells with high HER2 expression or Hs746T cells with low HER2 expression were co-cultured with PBMCs to compare PBMC-mediated cytotoxic effects. Indirect anti-cancer efficacy through immune activation was measured.

As shown in FIGS.8a and 8b, trastuzumab-IFNβ mutein exhibited cytotoxic efficacy through immune cell activation against NCI-N87 cells as well as Hs746T cells with a low HER2 expression level, proving that it has even better cancer cell cytotoxicity compared with trastuzumab.

Accordingly, it can be understood that trastuzumab-IFNβ mutein exerts a therapeutic effect on HER2-positive cancer even when the HER2 expression level is relatively low in contrast to trastuzumab which is effective only when the HER2 expression level is very high.

## Claims

1. A pharmaceutical composition for preventing or treating a cancer in which a HER2 expression level is IHC 1+ or higher, comprising a recombinant protein comprising an interferon-beta; and a HER2-targeting antibody or a fragment thereof covalently linked directly or indirectly to the interferon-beta as an active component.

2. The pharmaceutical composition of claim 1, wherein the interferon-beta is a variant of the interferon-beta of SEQ ID NO:1 in which the 27^{th} amino acid residue is substituted with threonine.

3. The pharmaceutical composition of claim 1, wherein the antibody or the fragment thereof is trastuzumab or pertuzumab.

4. The pharmaceutical composition of claim 1, wherein the recombinant protein is a protein in which the interferon-beta and the antibody or the fragment thereof are connected by a peptide linker.

5. The pharmaceutical composition of claim 1, wherein the cancer in which the HER2 expression level is IHC 1+ or higher is breast cancer or gastric cancer.

6. The pharmaceutical composition of claim 1, wherein the cancer expresses HER2 at the level of IHC 1+, or IHC 2+ in combination with negative FISH.

7. Use of a recombinant protein comprising an interferon-beta; and a HER2-targeting antibody or a fragment thereof covalently linked directly or indirectly to the interferon-beta to prepare a therapeutic agent for a cancer with a HER2 expression level of IHC 1+ or higher.

8. A method of treating a cancer in which a HER2 expression level is IHC 1+ or higher, comprising administering an effective amount of a composition to a subject in need thereof, the composition protein comprising an interferon-beta; and a HER2-targeting antibody or a fragment thereof covalently linked directly or indirectly to the interferon-beta as an active component.

9. A pharmaceutical composition for preventing or treating a cancer in which a HER2 expression level is IHC 1+ or higher, comprising an interferon-beta; and a HER2-targeting antibody or a fragment thereof as active components.

10. The pharmaceutical composition of claim 9, wherein the interferon-beta is a variant of the interferon-beta of SEQ ID NO:1 in which the 27^{th} amino acid residue is substituted with threonine.

11. The pharmaceutical composition of claim 9, wherein the interferon-beta; and the HER2-targeting antibody or the fragment thereof are formulated as a single composition or separate compositions.

12. The pharmaceutical composition of claim 9, wherein the interferon-beta; and the HER2-targeting antibody or the fragment thereof are administered simultaneously, separately or sequentially.

13. Use of a composition comprising an interferon-beta; and a HER2-targeting antibody or a fragment thereof to prepare a therapeutic agent for cancer in which a HER2 expression level is IHC 1+ or higher.

14. A method of treating a cancer in which a HER2 expression level is IHC 1+ or higher, comprising administering an effective amount of a composition to a subject in need thereof, the composition comprising an interferon-beta; and a HER2-targeting antibody or a fragment thereof.
